Europäisches Patentamt

European Patent Office

Office européen des brevets

(11) Veröffentlichungsnummer: **0 246 460 B1**

(12) **EUROPÄISCHE PATENTSCHRIFT**

(45) Veröffentlichungstag der Patentschrift: **18.12.91**

(51) Int. Cl.⁵: **G01R 33/58**, G01R 33/028, G01R 29/08

(21) Anmeldenummer: **87105891.3**

(22) Anmeldetag: **22.04.87**

(54) Verfahren zur Messung elektrischer oder magnetischer Wechselfelder und Anordnung zur Durchführung des Verfahrens.

(30) Priorität: **05.05.86 DE 3615197**

(43) Veröffentlichungstag der Anmeldung:
**25.11.87 Patentblatt 87/48**

(45) Bekanntmachung des Hinweises auf die Patenterteilung:
**18.12.91 Patentblatt 91/51**

(84) Benannte Vertragsstaaten:
**DE FR GB**

(56) Entgegenhaltungen:
**EP-A- 0 173 130**
**DE-A- 2 951 537**
**GB-A- 857 459**
**US-A- 3 777 189**
**US-A- 4 102 195**

**ANTENNAS AND PROPAGATION SOCIETY NEWSLETTER, Band 26, Nr. 5, Oktober 1984, Seiten 5-9, New York, US; J.C. WYSS: "Photonic sensors for electromagnetic field measurements"**

(73) Patentinhaber: **SIEMENS AKTIENGESELL-SCHAFT**
**Wittelsbacherplatz 2**
**W-8000 München 2(DE)**

(72) Erfinder: **Dürr, Wilhelm, Dr.**
**Damaschkestrasse 99**
**W-8520 Erlangen(DE)**
Erfinder: **Oppelt, Ralph, Dr.**
**Lindenweg 1**
**W-8525 Uttenreuth/Weiher(DE)**

EP 0 246 460 B1

RADIOLOGY, Band 149, Nr. 3, 1983, Seite 855, Easton, US; J.H. ELLIS et al.: "Communication device for patients undergoing nuclear magnetic resonance imaging"

INSTRUMENTS AND EXPERIMENTAL TECHNIQUES, Band 26, Nr. 4, Teil 2, Juli-August 1983, Seiten 955-956, Plenum Publishing Corp., New York, US; V.D. VOLOVIK et al.: "Electroacoustic magnetometer"

IEEE TRANSACTIONS ON POWER APPARATUS AND SYSTEMS, Band PAS-98, Nr. 2, März-April 1979, Seiten 449-455, IEEE, New York, US; R.T. HARROLD: "Acoustic waveguides for sensing and locating electrical discharges in high voltage power transformers and other apparatus"

## Beschreibung

Die Erfindung bezieht sich auf ein Verfahren zur Messung elektrischer oder magnetischer Wechselfelder, insbesondere zur Messung hochfrequenter Wechselfelder in Kernspin-Resonatoren.

Es ist bekannt, daß man die an Wasser gebundenen Wasserstoffatomkerne, d.h. Protonen, eines Untersuchungsobjektes aus einer Vorzugsrichtung, die durch ein magnetisches Grundfeld hoher statischer Feldstärke erzeugt wird, durch hochfrequente Anregungsimpulse zur Präzession anregen kann. Nach dem Ende eines Anregungsimpulses präzedieren die Atomkerne mit einer Frequenz, die von der Stärke des Grundfeldes abhängt und pendeln sich dann aufgrund ihres Spins nach einer vorbestimmten Relaxationszeit wieder in die Vorzugsrichtung ein. Durch rechnerische oder meßtechnische Analyse der integralen Protonensignale kann aus der räumlichen Spinndichte oder der Verteilung der Relaxationszeiten innerhalb einer Körperschicht ein Bild erzeugt werden. Die Zuordnung des infolge der Präzessionsbewegung nachweisbaren Kernresonanzsignals zum Ort seiner Entstehung erfolgt durch die Anwendung linearer Feldgradienten. Diese Gradientenfelder werden dem Grundfeld überlagert und so gesteuert, daß nur in der abzubildenden Schicht eine Anregung der Protonen erfolgt. Diese Bilddarstellung ist bekannt unter den Bezeichnungen Kernspin-Tomographie KST, NMR-Tomographie (nuclear magnetic resonanz), Zeugmatographie oder Spin-Imaging sowie Spin-Mapping.

Die Qualität der erzeugten Schnittbilder wird wesentlich bestimmt vom Signal-Rauschverhältnis des induzierten Kernspin-Resonanzsignals. Da dieses wieder vom Grundfeld abhängt und mit der Frequenz steigt, sind bei hohen Grundfeldern auch hohe Frequenzen vorgesehen. Weitgehend homogene hochfrequente Magnetfelder mit hohem Signal-Rauschverhältnis zur Signalanregung und -aufnahme können beispielsweise von Hochfrequenzspulen erzeugt werden, denen eine gemeinsame weitgehend hochfrequenzdichte und niederfrequenzdurchlässige Hülle aus elektrisch gut leitendem Material zugeordnet sein kann. Im gesamten von den Hochfrequenzspulen eingeschlossenen Volumen, in welches das Untersuchungsobjekt eingeschoben wird, entstehen gleichphasig schwingende Felder (DE-OS 31 33 432).

Hierzu ist jedoch erforderlich, daß das Hochfrequenzmagnetfeld homogen ist. Es muß somit das vorhandene Hochfrequenzmagnetfeld vorher mittels einer Meßsonde in Abhängigkeit von der Sondenposition ermittelt werden. Um Meßfehler zu begrenzen, darf das zu messende Hochfrequenzmagnetfeld durch die Meßeinrichtung nicht gestört werden. Die örtliche Ausdehnung der metallischen Komponenten der Meßeinrichtung muß somit klein gehalten werden. Insbesondere darf zur mechanischen Halterung der Sonde kein metallischer Träger und zur Signalauslese keine metallische elektrische Leitung, beispielsweise kein Koaxkabel, verwendet werden.

Der Erfindung liegt somit die Aufgabe zugrunde, ein Verfahren zur Messung elektrischer oder magnetischer Felder sowie eine Anordnung zur Durchführung dieses Verfahrens anzugeben, das mit geringem Aufwand eine genaue vektorielle Messung der Felder nach Betrag und Richtung ohne wesentliche Störung dieser Felder ermöglicht. Die zur Halterung der Sonden verwendeten Materialien sollen möglichst die gleichen dielektrischen und magnetischen Eigenschaften haben wie das Medium im Meßraum.

Es sind Sonden zur Messung elektrischer oder magnetischer Felder bekannt, bei denen die mechanische Halterung als starres Koaxkabel ausgebildet wird und zugleich als elektrische Leitung dient. Bedingt durch die zwangsweise große Längenausdehnung einer solchen metallischen Halterung bzw. Leitung wird durch das Einführen dieser Sonde in das zu messende Feld, beispielsweise einen Resonator, dieses ursprünglich zu messende hochfrequente elektromagnetische Feld gestört. Der damit verbundene Meßfehler wird in Kauf genommen.

Aus Dokument US-A-4 102 195 sind ein Verfahren und eine Anordnung zur Durchführung einer Messung innerhalb eines geschlossenen Meßraumes bekannt, wobei durch eine akustische Übertragungstrecke ein akustischer Resonator innerhalb des Raumes mit einem Signalempfänger außerhalb des Raumes verbunden wird.

Ferner sind Sonden zur Messung von Antennennahfeldern bekannt, bei denen die Signalauslese optisch über Lichtleitfasern erfolgt ("Mikrowaves & RF", Juni 1985, Seiten 51 bis 55). Zur Umsetzung der gemessenen elektrischen Signale in optische Signale ist aber ein verhältnismäßig aufwendiges Interface mit eingebauter Stromversorgung erforderlich, dessen metallische Ausdehnung in mindestens einer Dimension mehrere Zentimeter beträgt. Diese optische Signalauslese ist somit beispielsweise für Resonatormessungen ungeeignet.

Die Lösung der genannten Aufgabe besteht nun erfindungsgemäß in den Verfahrensmerkmalen des Anspruchs 1. Die Ultraschall-Übertragungsstrecke enthält keine metallischen Teile und die Feldstörung durch diese Meßeinrichtung ist somit entsprechend gering. Die Ausdehnung der erforderlichen Meßwertaufnehmer mit dem zugeordneten Ultraschall-Sendewandler ist begrenzt auf wenige Millimeter.

Zur Messung von Feldern mit sehr hoher Frequenz kann eine Modulation des Feldes zweckmäßig sein. Dann wird gemäß einer vorteilhaften weiteren Ausgestaltung des Verfahrens das hochfrequente elektromagnetische Feld mit einem niederfrequenten Meßsignal, auf dem die Signalübertragungsstrecke arbeitet, amplitudenmoduliert und die Meßsonde ist unabhängig von der Frequenz des zu messenden Feldes und kann somit für einen großen Frequenzbereich eingesetzt werden.

Die von der Meßsonde verursachte Feldstörung ist praktisch vernachlässigbar. Das Verfahren kann deshalb vorzugsweise verwendet werden in Resonatoren oder auch im Nahbereich von Antennen. Ferner ist es geeignet zur vektoriellen Vermessung von elektromagnetischen Feldern. Eine Schleifenausführung der Meßsonde kann eine Vektorkomponente des magnetischen Feldes, eine Ausführung mit einem kleinen Stabdipol eine Vektorkomponente des elektrischen Feldes messen. Nach Drehung der Meßsonde können die übrigen Feldkomponenten erfaßt werden.

In einer Anordnung zur Durchführung des Verfahrens ist dem Signalaufnehmer ein Ultraschall-Sendewandler zugeordnet und als Übertragungsstrecke ist ein nichtmetallischer akustischer Wellenleiter vorgesehen. Am Ende der Übertragungsstrecke ist ein Ultraschall-Empfangswandler angeordnet, der das übertragene Schallsignal wieder in ein entsprechendes elektrisches Signal umwandelt.

Zur weiteren Erläuterung der Erfindung wird auf die Zeichnung Bezug genommen, in deren Figur 1 eine Anordnung zur Feldmessung in einem Kernspin-Hochfrequenzresonator schematisch veranschaulicht ist. In Figur 2 ist ein Ausführungsbeispiel einer Signalübertragungsstrecke dargestellt. Die Figuren 3 bis 5 zeigen jeweils eine Ausführungsform eines Meßwertaufnehmers.

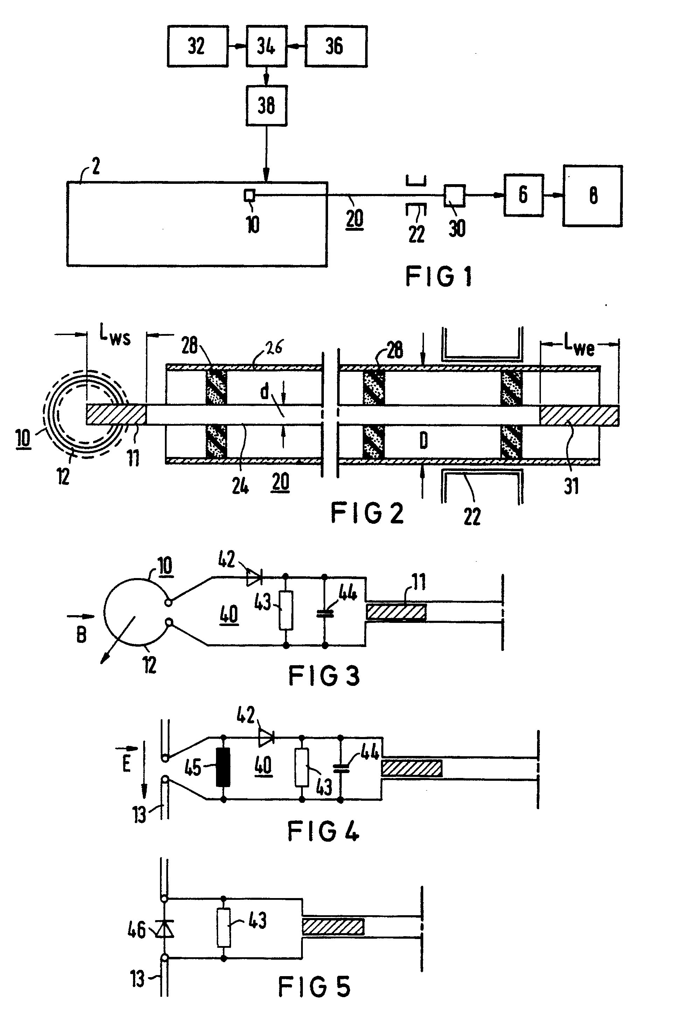

Zum Herstellen von Schnittbildern eines Meßobjekts mit einem Kernspintomographen ist es erforderlich, eine Hochfrequenzspule 2 gemäß Figur 1 so auszubilden, daß in ihrem Innenraum ein möglichst homogenes Feld entsteht. Zum Ausmessen dieses Feldes ist deshalb eine Meßeinrichtung mit einem Signalaufnehmer 10, der einen Ultraschall-Sendewandler enthält, und einer Übertragungsstrecke 20 mit einem nichtmetallischen akustischen Wellenleiter sowie einem Signalempfänger 30 vorgesehen, der einen Ultraschall-Empfangswandler enthält. Die Übertragungsstrecke 20 ist in einer Halterung 22 verschiebbar eingespannt und ragt freitragend in den Meßraum der Hochfrequenzspule 2 hinein.

In einer bevorzugten Ausführungsform einer Anordnung zur Durchführung des Verfahrens erhält nun die Hochfrequenzspule 2 ein amplitudenmoduliertes Hochfrequenzsignal. Zu diesem Zweck ist ein Hochfrequenzgenerator 32, beispielsweise für eine Frequenz von 85 MHz, vorgesehen, dessen Ausgangssignal durch einen Amplitudenmodulator 34 mit dem Signal eines Niederfrequenzgenerators 36 mit einer Frequenz von beispielsweise 101,3 KHz moduliert und der Hochfrequenzspule 2 zugeführt wird. Zur Signalverstärkung kann vorzugsweise noch ein Verstärker 38 vorgesehen sein. Dem Signalempfänger 30 ist dann ein schmalbandiges Filter 6 für die Frequenz des Niederfrequenzgenerators 36 sowie ein Auswertegerät 8 nachgeschaltet, das beispielsweise ein Voltmeter sein kann.

In der Ausführungsform einer Übertragungsstrecke 20 gemäß Figur 2 wird das aufgenommene Meßsignal vom Signalaufnehmer 10, von dem lediglich der Ultraschall-Sendewandler 11 mit einer Meßschleife 12 dargestellt ist, umgewandelt in ein entsprechendes Ultraschallsignal. Das Ultraschallsignal wird über einen Schallwellenleiter 24 zum Signalempfänger geleitet, von dem lediglich der Ultraschall-Empfangswandler 31 in der Figur angedeutet ist, dessen Länge $L_{we}$ beispielsweise 22 mm betragen kann. Der Schallwellenleiter 24 mit einer Dicke d von beispielsweise 2 mm ist von einem Außenrohr 26 mit einer Dicke D von beispielsweise 6 mm und einer Wandstärke von beispielsweise 1 mm konzentrisch umgeben, das mit Hilfe von Schallisolatoren 28 gegenüber dem Schallwellenleiter 24 akustisch isoliert ist. Der stabförmige Schallwellenleiter 24 besteht aus einem Material hoher akustischer Güte und niedriger Dielektrizitätszahl, beispielsweise Glas oder Plexiglas, vorzugsweise Quarzglas. Der Durchmesser des Schallwellenleiters 24 wird wesentlich bestimmt von der freitragenden Länge, d.h. dem Abstand des Signalaufnehmers 10 von der Einspannvorrichtung 22. Das Außenrohr 26 dient als Tragkörper und besteht somit aus einem Material hoher mechanischer Festigkeit und geringer Dielektrizitätskonstante, vorzugsweise Quarzglas oder auch Kunststoff. Die als Schallisolatoren 28 vorgesehenen Distanzringe bestehen aus einem schallweichen Medium, beispielsweise Schaumstoff.

Die Ultraschall-Signale werden mit dem akustischen Schallwellenleiter 24 der Übertragungsstrecke 20, der zugleich als Sondenhalterung dient, aus der Hochfrequenzspule 2 geleitet und dann mit dem Ultraschall-Empfangswandler 31 wieder in elektrische Signale umgewandelt. Aufgrund der endlichen Länge des Schallwellenleiters 24 wirkt dieser im CW-Betrieb als akustischer Resonator. Sein longitudinaler Schwingungsmodenabstand, d.h. der Frequenzabstand seiner Eigenresonanzen, ist durch Länge und Schallgeschwindigkeit des Schallwellenleitermaterials gegeben. Die exakte Länge des Schallwellenleiters 24 wird zur Erzielung hoher Empfindlichkeit vorzugsweise so gewählt, daß eine seiner Eigenresonanzfrequenzen mit der Frequenz des zu messenden elektromagnetischen Wechselfeldes übereinstimmt. Auch die Eigenresonanz der beiden Ultraschallwandler kann vorzugsweise mit dieser Frequenz wenigstens annähernd übereinstimmen.

In der Ausführungsform eines Signalaufnehmers 10 gemäß Figur 2 zur Messung magnetischer Felder sind die Enden der Meßschleife 12, die vorzugsweise noch mit einer in der Figur gestrichelt angedeuteten Abschirmung versehen sein kann, mit den nicht näher bezeichneten Elektroden des Ultraschall-Sendewandlers 11 verbunden, dessen Länge $L_{ws}$ beispielsweise 7 mm betragen kann und der das aufgenommene elektrische Signal in ein entsprechendes akustisches Signal mit gleicher Frequenz umwandelt und mit dem Schallwellenleiter 24 zum Signalempfänger 30 überträgt.

Da diese Meßeinrichtung durch das Material und die Dimensionierung der Ultraschallwandler nur in einem vorbestimmten Frequenzbereich die Signale gut überträgt, d.h. verhältnismäßig schmalbandig arbeitet, kann in der besonderen Ausführungsform der Anordnung zur Durchführung des Verfahrens gemäß Figur 1 eine Hochfrequenzmodulation des Anregungssignals der Hochfrequenzspule 2 und damit des Meßsignals vorgesehen sein. Dann ist der Signalaufnehmer 10 gemäß Figur 3 mit einem entsprechenden Demodulator 40 versehen. In dieser Ausführungsform der Meßeinrichtung, insbesondere zum Messen sehr hochfrequenter Wechselfelder, wird das Meßsignal mit der wesentlich geringeren Frequenz, beispielsweise im Bereich von etwa 10 kHz bis 1 MHz, vorzugsweise etwa 100 kHz, moduliert und dann demoduliert. Der Demodulator 40 entnimmt aus dem Hochfrequenzsignal die Modulationsfrequenz. Übertragen wird somit nur noch das niederfrequente Modulationssignal, das in seiner Amplitude proportional ist dem gemessenen Hochfrequenzfeld. Ist die optimale Übertragungsfrequenz der Anordnung beispielsweise mit 101,3 kHz ermittelt, so wird die eingespeiste Frequenz der Hochfrequenzspule 2 genau mit dieser Frequenz amplitudenmoduliert. Es können somit HF-Felder nahezu beliebiger Frequenz mit der gleichen Meßeinrichtung gemessen werden.

In Verbindung mit der Stromschleife 12 als magnetischer Dipol zur Messung der magnetischen Induktion B kann beispielsweise ein passiver Mischer vorgesehen sein, der eine Diode 42 und einen Widerstand 43 mit beispielsweise 200 Kiloohm sowie gegebenenfalls einen Kondensator 44 enthält, wenn die Eigenkapazität des Ultraschall-Sendewandlers 11 zur einwandfreien Demodulation nicht ausreicht. Ein Signalempfänger 10 in dieser Ausführungsform hat mit seinem Ultraschall-Sendewandler 11 nur eine geringe Ausdehnung, die wenige Millimeter nicht wesentlich überschreitet. Eine Feldstörung durch diesen Signalempfänger 10 ist somit praktisch ausgeschlossen.

Gemäß Figur 4 kann in Verbindung mit einem elektrischen Dipol 13 zur Messung der elektrischen Feldstärke der Demodulator 40 eine Induktivität 45 enthalten, welche die nicht näher bezeichneten Pole des Dipols 13 überbrückt.

In der Ausführungsform eines Signalaufnehmers 10 gemäß Figur 5 mit dem elektrischen Dipol 13 enthält der Demodulator 40 ebenfalls eine Diode 46, die im Dipol 13 angeordnet ist und der ein Parallelwiderstand 43 zugeordnet ist.

Anstelle dieser passiven Demodulationseinrichtungen 40 gemäß den Figuren 3 bis 5 können gegebenenfalls auch aktive Mischer vorgesehen sein, insbesondere zur Messung verhältnismäßig schwacher magnetischer und elektrischer Felder. Solche aktiven Mischer benötigen jedoch eine zusätzliche Stromversorgung, beispielsweise eine Batterie, die das Volumen des Signalaufnehmers 10 entsprechend vergrößert.

## Patentansprüche

1. Verfahren Zur Übertragung eines aus der Ausmessung eines elektrischen oder magnetischen Wechselfeldes abgeleiteten Meßsignals, insbesondere bei der Ausmessung hochfrequenter Wechselfelder in Kernspin-Tomographen, **dadurch gekennzeichnet,** daß das Meßsignal durch einen magnetischen oder elektrischen Dipol (12, 13) erzeugt wird, daß das Meßsignal in ein entsprechendes Ultraschallsignal umgewandelt wird, das zu einem Empfänger (30) übertragen und dann wieder in ein elektrisches Signal umgewandelt wird.

2. Verfahren nach Anspruch 1 für hochfrequente Wechselfelder, **gekennzeichnet** durch eine Modulation des Anregungssignals für das hochfrequente Wechselfeld und durch Demodulation des Meßsignals und Übertragung des niederfrequenten Demodulationssignals, dessen Amplitude dem Meßsignal proportional ist.

3. Anordnung zur Durchführung des Verfahrens nach Anspruch 1 oder 2 mit einem Signalaufnehmer und einer Übertragungsstrecke sowie einem Signalempfänger, **dadurch gekennzeichnet,** daß dem Signalaufnehmer (10) mit dem magnetischen oder elektrischen Dipol (12, 13) ein UltraschallSendewandler (11) zugeordnet ist und daß als Übertragungsstrecke (20) ein nichtmetallischer Schallwellenleiter (24) vorgesehen ist und daß am Ende der Übertragungsstrecke (20) ein Ultraschall-Empfangswandler (31) angeordnet ist.

4. Anordnung nach Anspruch 3, **dadurch gekennzeichnet,** daß dem Signalaufnehmer (10) eine Demodulationseinrichtung (40) zugeordnet ist.

**5.** Anordnung nach Anspruch 3 oder 4, **dadurch gekennzeichnet,** daß als Übertragungsstrecke (20) ein Schallwellenleiter (24) aus einem Material mit hoher akustischer Güte und niedriger Dielektrizitätszahl vorgesehen ist, der koaxial in einem Außenrohr (26) aus einem Material mit hoher mechanischer Festigkeit und geringer Dielektrizitätskonstante angeordnet ist, und daß zwischen dem Schallwellenleiter (24) und dem Außenrohr (26) Schallisolatoren (28) angeordnet sind.

**6.** Anordnung nach Anspruch 5, **dadurch gekennzeichnet,** daß als Schallisolatoren (28) Distanzringe aus Schaumstoff vorgesehen sind.

## Claims

**1.** Method for transmitting a measurement signal which is derived from the measurement of an electric or magnetic alternating field, in particular in the case of measurement of high-frequency alternating fields in nuclear spin tomographs, characterised in that the measurement signal is generated by a magnetic or electric dipole (12, 13), and in that the measurement signal is converted into a corresponding ultrasonic signal which is transmitted to a receiver (30) and then is converted back into an electrical signal.

**2.** Method according to claim 1 for high-frequency alternating fields, characterised by modulation of the excitation signal for the high-frequency alternating field and by demodulation of the measurement signal and transmission of the low-frequency demodulation signal, the amplitude of which is proportional to the measurement signal.

**3.** Arrangement for carrying out the method according to claim 1 or 2 having a signal sensor and a transmission section and also a signal receiver, characterised in that an ultrasonic transmitting transducer (11) is associated with the signal sensor (10) having the magnetic or electric dipole (12, 13) and in that a non-metallic sound-wave conductor (24) is provided as a transmission section (20) and in that an ultrasonic receiving transducer (31) is arranged at the end of the transmission section (20).

**4.** Arrangement according to claim 3, characterised in that a demodulation device (40) is associated with the signal sensor (10).

**5.** Arrangement according to claim 3 or 4, characterised in that there is provided as a transmission section (20) a sound-wave conductor (24) which consists of a material with high acoustic quality and low dielectric coefficient and which is arranged coaxially in an outer tube (26) of a material with high mechanical strength and low dielectric constant, and in that sound insulators (28) are arranged between the sound-wave conductor (24) and the outer tube (26).

**6.** Arrangement according to claim 5, characterised in that spacer rings of foamed material are provided as sound insulators (28).

## Revendications

**1.** Procédé pour transmettre un signal de mesure tiré de la mesure d'un champ alternatif électrique ou magnétique, notamment lors de la mesure de champ alternatif à haute fréquence dans des tomographes à résonance magnétique nucléaire, caractérisé par le fait que le signal de mesure est produit par un dipôle magnétique ou électrique (12,13), que le signal de mesure est converti en un signal ultrasonore correspondant, qui est envoyé à un récepteur (30) et est à nouveau converti en un signal électrique.

**2.** Procédé suivant la revendication 1 pour des champs alternatif à haute fréquence, caractérisé par une modulation du signal d'excitation pour le champ alternatif à haute fréquence, et par une démodulation du signal de mesure et la transmission du signal démodulé à fréquence inférieure, dont l'amplitude est proportionnelle au signal de mesure.

**3.** Dispositif pour la mise en oeuvre du procédé suivant la revendication 1 ou 2, comportant un capteur de signaux et une section de transmission ainsi qu'un récepteur de signaux, caractérisé par le fait qu'un transducteur d'émission à ultrasons (11) est associé au capteur de signaux (10) comportant le dipôle magnétique ou électrique (12,13) et qu'un guide non métallique d'ondes acoustiques (24) est prévu en tant que section de transmission (20) et qu'un transducteur de réception d'ultrasons (31) est disposé à l'extrémité de la section de transmission (20).

**4.** Dispositif suivant la revendication 3, caractérisé par le fait qu'un dispositif de démodulation (40) est associé au capteur de signaux (10).

**5.** Dispositif suivant la revendication 3 ou 4, caractérisé par le fait qu'il est prévu, comme section de transmission (20), un guide d'ondes acoustiques (24) réalisé en un matériau possédant une qualité acoustique élevée et une constante diélectrique faible et qui est disposé coaxialement dans un tube extérieur (26) réalisé en un matériau possédant une grande résistance mécanique et une faible constante diélectrique, et que des isolateurs acoustiques (28) sont disposés entre le guide d'ondes acoustiques (24) et le tube extérieur (26).

6. Dispositif suivant la revendication 5, caractérisé par le fait que des bagues-entretoises en matériau mousse sont prévues en tant qu'isolateurs acoustiques (28).

FIG 1

FIG 2

FIG 3

FIG 4

FIG 5